# EUROPEAN PATENT APPLICATION

(11) **EP 0 595 715 A1**
(43) Date of publication of application: **04.05.1994**
(21) Application number: 93402632.9
(22) Date of filing: 27.10.1993
(51) Int. Cl.: A61K 33/44

(54) **Activated carbon as antinephrotic syndrome agent**

(30) Priority: 29.10.1992 JP 314180/92
(71) Applicant: KUREHA CHEMICAL INDUSTRY CO., LTD., Chuo-ku Tokyo 103 (JP)
(72) Inventor: Ise, Michihito, Kawagoe-shi, Saitama-ken 350 (JP); Sugano, Mikio, 201 Kureha Kagaku Okubo Shataku, Shinjuku-ku, Tokyo 169 (JP)
(74) Representative: Portal, Gérard

(57) **Abstract**

The present invention provides an antinephrotic syndrome agent containing a spherical carbon as an active ingredient in an amount effective for treating nephrotic syndrome. The agent is orally administered to patients and , unlike conventional steroid medicines, has no side effects. Further, the present invention provides a method of treating nephrotic syndrome including the step of administering an effective amount of spherical carbon to a patient.

## Description

### BACKGROUND OF THE INVENTION

### (1) Field of the Invention

The present invention relates to an antinephrotic syndrome agent comprising a spherical carbon as an active ingredient.

### (2) Description of the Related Art

Nephrotic syndrome is caused by various diseases with common clinical symptoms such as proteinuria, etc.

The main symptom of the nephrotic syndrome is persistent proteinuria which shows urinary protein in a large amount of 3.5 g/day or more. The prevalence of nephrotic syndrome is estimated to be about 30 infants and about 15 adults per 100,000 population. Before medicines such as diuretics, antibiotics, and adrenal corticosteroids appeared, the prognosis was not favorable due to a severe edema or a complication of an infectious disease. After these medicines appeared, however, the prognosis has improved to the extent that more than 90% of patients survive for 10 years.

Heretofore, glucocorticoid has been used as the most effective medicine for the nephrotic syndrome. In principle, prednisolone is used in a large amount of 30 to 40 mg/day in an early stage, and maintenance therapy is then continued using prednisolone in an amount of 20 mg/48 hr after the symptoms are alleviated. In treatment of the nephrotic syndrome, prednisolone is administered in the form of an oral medicine such as a bulk powder, a powder, or a tablet, and a prednisolone derivative is administered in the form of an intravenous injection, intravenous drip, or intramuscular injection.

Although the symptoms can easily be remitted in an early stage by conventional steroid therapy, the relapse rates of infant and adult patients are as high as 40 to 90% and 30 to 50%, respectively. Further, the administration of prednisolone or derivatives thereof sometimes causes severe side effects such as induced infectious disease, secondary adrenocortical insufficiency, peptic ulcer, diabetes, psychopathy, or steroid nephropathy.

### SUMMARY OF THE INVENTION

It is an object of the present invention to provide an antinephrotic syndrome agent comprising a spherical carbon as an active ingredient which has no side effects.

It is another object of the present invention to provide a method of treating nephrotic syndrome without side effects.

The inventors of the present invention performed extensive research for obtaining an active ingredient which is different from conventional steroid compounds.

As a result, the inventors discovered that the oral administration of a spherical carbon to rats suffering from nephrotic syndrome decreases the urinary protein. The present invention is based on those findings.

In order to achieve these objects, the present invention provides an antinephrotic syndrome agent comprising a substantially spherical carbon as an active ingredient in an amount effective for treating nephrotic syndrome.

According to a particular embodiment, said spherical carbon comprises an activated spherical carbon.

According to another particular embodiment, said spherical carbon comprises a spherical carbonaceous adsorbent.

According to another particular embodiment, the spherical carbon has a particle diameter ranging from 0.05 to 2 mm.

According to a further particular embodiment, said spherical carbon, said activated spherical carbon or said spherical carbonaceous adsorbent has a pore radius of less than 80 angströms in a pore amount of 0.2 to 1.0 ml/g, a total amount of acidic groups (A) of 0.30 to 1.20 m eq/g, a total amount of basic groups (B) of 0.20 to 0.70 m eq/m and a ratio of the total amount of acidic groups (A)/ total amount of basic groups (B) of 0.40 to 2.5.

The present invention further provides a pharmaceutical composition against nephrotic syndrome, characterized in that it comprises an anti-nephrotic syndrome agent as previously defined, in a dosage form, optionnaly in combination with a pharmaceutically acceptable excipient or vehicle.

The present invention again provides a pharmaceutical composition against nephrotic syndrome, charaterized in that it comprises a substantially spherical carbon as it is in a dosage unit form.

In a particular embodiment, the dosage unit form is a capsule, a stick package or a divided package. By this way, it may be possible to manufacture the spherical carbon or the spherical carbonaceous adsorbent for instance in the form of a capsule containing the carbon or adsorbent alone and which is preferably used as such for the treatment of the nephrotic syndrome.

The present invention further provides a method of treating nephrotic syndrome comprising administering an effective amount of spherical carbon to a patient in need thereof.

### DETAILED DESCRIPTION OF THE PREFERRED EMBODIMENT

A spherical carbon used as an active ingredient in the present invention is not particularly limited as long as the spherical carbon comprises activated carbon particles having a substantially spherical shape which can be used for medical treatment. Although a medical activated carbon powder is generally useful as an antidote, it is liable to cause constipation as a side effect. This is a critical problem because constipation at the time of illness is dangerous.

The spherical carbon used in the present invention has a particle diameter ranging from 0.05 to 2 mm. When the diameter is less than 0.05 mm, side effects such as constipation or the like are not sufficiently eliminated. On the other hand, when the diameter is over 2mm, oral administration of the spherical carbon becomes difficult, and the desired pharmacological effect does not appear quickly.

The shape of the spherical carbon is an important factor for obtaining satisfactory medical effects of the present invention, and it is necessary that the spherical carbon has a substantially spherical shape.

Any raw materials for activated carbon may be used for producing the spherical carbon of the present invention. Although examples of such raw materials that can be used includes sawdust, coal, coconut-shell, petroleum pitches, coal pitches, and synthetic organic high polymers, petroleum hydrocarbons are preferable. In the present invention, it is preferable to use an activated spherical carbon and/or a spherical carbonaceous adsorbent as the spherical carbon.

Particles of an activated spherical carbon which can be used in the present invention have a diameter of 0.05 to 2 mm.

A fundamental method of producing the activated spherical carbon according to the present invention comprises forming a raw material into fine spherical particles, carbonizing the spherical particles, and then activating the carbonized particles.

Various activation methods can be carried out, for example, using water vapor, chemicals, air, or carbon dioxide;

The activated spherical carbon particles can be produced, for example, by the following three methods. A first method comprises forming a raw material powder into fine spherical particles by using a binder such as pitch, carbonizing the thus-formed particles by baking the particles in an inert atmosphere at 600 to 1000°C, and then activating the carbonized particles in an atmosphere of steam at 850 to 1000°C. A second method comprises forming molten pitch into fine spherical particles, oxidizing the particles in an atmosphere containing oxygen to render the particles infusible; and then carbonizing and activating the infusible particles under the same conditions as those in the first method above, as disclosed in, for example, Japanese Patent Publication no. 51-76 (see US Patent no. 3,917,806). A third method comprises melt-extruding pitch to form a string-like pitch, breaking the string-like pitch, casting the broken product into hot water to obtain spherical particles, oxidizing the thus-obtained particles in an atmosphere containing oxygen to render the particles infusible, and then carbonizing and activating the infusible particles under the same conditions as those in the first method above, as disclosed in, for example, Japanese Patent Publication no. 59-10930 (see US Patent No. 4,420,443).

The spherical carbonaceous adsorbent which can be used in the present invention preferably comprises activated carbon particles having a diameter of 0.05 to 2 mm, a pore radius of less than 80 angstroms in a pore amount of 0.2 to 1.0 ml/g, a total amount of acidic groups (A) of 0.30 to 1.20 meq/g, a total amount of basic groups (B) of 0.20 to 0.70 meq/g, and a ratio of the total amount of acidic groups (A) / total amount of basic groups (B) of 0.40 to 2.5. An example of these spherical carbonaceous adsorbents is disclosed in Japanese Patent Publication No. 62-11611 (see the specification of US patent No.4,681,764).

The spherical carbonaceous adsorbent can be produced by oxidizing and reducing, at a high temperature, activated spherical carbon particles having a diameter of 0.05 to 2 mm and a pore radius of less than 80 angstroms in a pore amount of 0.2 to 1.0 ml/g. Oxidation and reduction at a high temperature are preferably made so that the total amount of acidic groups (A) and the total amount of basic groups (B) of the spherical carbonaceous adsorbent obtained are adjusted within the ranges of 0.30 to 1.20 meq/g and 0.20 to 0.70 meq/g, respectively, and the ratio of (A) / (B) is adjusted within the range of 0.40 to 2.5

The total amount of acidic groups (A) and the total amount of basic groups (B) are determined by the following usual methods.
(a) Total amount of acidic groups (A)
   One gram of pulverized adsorbent specimen which passed through a Taylor standard sieve of 200 mesh is added to 50 ml of a 0.05 N aqueous Na0H solution, followed by shaking for 48 hours. The resultant mixture is filtered to remove the adsorbent, and the thus-obtained filtrate is neutralized by titration. The total amount of acidic groups (A) is determined by the amount of Na0H consumed by the titration and is expressed in the units of meq/g of specimen.
(b) Total amount of basic groups (B)
   One gram of pulverized adsorbent specimen which passed through a Taylor standard sieve of 200 mesh is added to 50 ml of a 0.05 N aqueous HC1 solution, followed by shaking for 24 hours. The resultant mixture is filtered to remove the specimen, and the thus-obtained filtrate is neutralized by titration.

The total amount of basic groups (B) is determined by the amount of HC1 consumed by the titration and is expressed in the units of meq/g of specimen.

High temperature oxidation is performed by heating the particles at a high temperature in an atmosphere containing oxygen, which is formed by using pure oxygen, nitrogen oxides or air as an oxygen source.

High temperature reduction is performed by heating the particles at a high temperature in an atmosphere of a gas inert against carbon. The atmosphere of a gas inert against carbon is formed by using nitrogen, argon, helium, or a mixture thereof.

Oxidative heating is preferably carried out at 300 to 700°C, more preferably 400 to 600°C, in an atmosphere containing preferably 0.5 to 25% by volume of oxygen, more preferably 3 to 10% by volume of oxygen.

Reduction is preferably carried out at 700 to 1100°C, more preferably 800 to 1000°C, in an atmosphere of nitrogen.

When the above spherical carbonaceous adsorbent was orally administered to rats which had been given puromycin aminonucleoside to cause nephrotic syndrome and which rats were known as models of nephrotic syndrome, the inventors found the surprising phenomenon that the urinary protein of the rats had been decreased. Therefore, a medicine containing the spherical carbon as an active ingredient is found to be an antinephrotic syndrome agent effective for nephrotic syndrome.

Further, when the antinephrotic syndrome agent of the present invention was administered to normal rats, no abnormality was induced.

The antinephrotic syndrome agent of the present invention can be administered orally. The dosage of the agent depends on the subject (animal or human), the age of the subject, differences among subjects, the conditions of the disease, etc. For example, the oral dosage of the spherical carbon for humans is within the range of 0.2 to 20 g per day. The dosage may be administered at one time or divided into 2 to 4 doses and administered. The dosage may be adjusted appropriately according to symptoms.

Thus, the spherical carbon can be administered as it is or in the form of a pharmaceutical composition as an antinephrotic syndrome agent.

The spherical carbon may be administered as a medicine to patients in any desired form such as granules, tablets, sugar-coated tablets, capsules, stick packages, divided packages, suspensions, or the like.

When the particles are administered in the form of capsules, ordinary gelatin capsules, or, if necessary, enteric capsules may be used. When the carbon particles are used in the form of granules, tablets, or sugar-coated tablets, the form must be disintegrated into the original fine spherical particles in the alimentary canal of a patient.

Although the content of the spherical carbon in a pharmaceutical composition may be varied according to symptoms and other factors, it is usually 1 to 99% by weight, preferably 10 to 99% by weight.

The antinephrotic syndrome agent of the present invention can be used for treating nephrotic syndrome by oral administration and, unlike conventional steroid medicines, has no side effects.

### EXAMPLES

Although the present invention will be more precisely explained below with reference to examples, the invention is not limited to these examples.

### Production Example 1: Preparation of spherical carbonaceous adsorbent

An autoclave equipped with a stirrer was charged with 100 g of naphthalene and 300 g of pitch (H/C=0.55, flow point = 220°c), having an anisotropic region which was not localized under a polarization microscope. The resultant mixture was mixed well at 180°C to form a solution. Into the resulting solution, 1200 g of 0.5 % aqueous polyvinyl alcohol solution was added. Then, the mixture was vigorously stirred at 140°C for 30 minutes and cooled to room temperature under stirring to form a dispersion of spherical particles. After a large part of water was separated from the dispersion, the remaining spherical particles were treated with hexane in an extractor to remove naphthalene contained therein by extraction and then dried by air flow. The thus-obtained particles were heated to 300°C at a rate of 25°C/hr by a flow of heated air in a fluidized bed system, and were further maintained for 2 hours at a fixed temperature of 300°C to obtain infusible oxygen-containing spherical particles. The thus-obtained particles were then heated to 900°C in steam and kept at 900°C for 2 hours in steam so as to carbonize and activate the particles to obtain porous activated spherical carbon. The activated spherical carbon had a diameter of 0.05 to 1.0 mm and a pore radius of less than 80 angstroms in a pore amount of 0.755 ml/g, which was determined by a methanol adsorption method using an automatic adsorption measuring apparatus.

The thus-obtained activated spherical carbon particles were heated to 600°C in an atmosphere containing 3 % by volume of oxygen, and were further heated for 3 hours in the same atmosphere using a fluidized bed. Then, the particles were further heated to 950°C in an atmosphere of nitrogen and kept at 950°C for 30 minutes in the same atmosphere to obtain an intended spherical carbonaceous adsorbent (hereinafter referred to as "Sample 1").

The thus-obtained spherical carbonaceous adsorbent has a diameter of 0.05 to 1 mm, a pore radius of less than 80 angstroms in a pore amount of 0.751 ml/g, which was determined by the methanol adsorption method using an automatic adsorption measuring apparatus, a total amount of acidic groups (A) of 0.542 meq/g, a total amount of basic groups (B) of 0.525 meq/g, and a ratio of the total amount of acidic groups (A) / total amount of basic groups (B) of 1.03.

In an acute toxicity test of the spherical carbonaceous adsorbent by oral administration to male and female rats (Cpb: WU: Wistar Random), no abnormality was observed even at the maximum dosage (5000 mg/kg for male and female rats) based on the Guidelines for Toxicity Studies of Drugs (Notification No. 118 of the Pharmaceutical Affairs Bureau, Ministry of Health and Welfare, Japanese Government, February 15, 1984).

### Example 1: Effect of antinephrotic syndrome agent on rats suffering from nephrotic syndrome

Sample 1 obtained in the above Production Example 1 was used as a spherical carbonaceous adsorbent which was an active ingredient of the antinephrotic syndrome agent.

Model rats for nephrotic syndrome were prepared by subcutaneously administering puromycin aminonucleoside (produced by Sigma Co., hereinafter referred to as "AN") to Jcl-SD male rats (Japan CLEA; body weight from 175 to 203 g) in a amount of 20 mg/kg of rat body weight, 7 times (0, 1, 2, 4, 6, 8, and 9, weeks after). AN was administered in the form of a 2% solution in physiological saline.

After AN was administered 7 times, i.e. after 9 weeks has passed, the urinary protein amounts of the rats reached maximum values. At this time, the rats were divided into two groups, a spherical carbonaceous adsorbent administration group (A group) and a control group (C group), so that there were no deviation in the body weights and the amounts of urinary protein between the two groups.

A standard group (S group) was prepared by subcutaneously administering 7 times physiological saline without AN to rats instead of the above 7 subcutaneous administrations of the physiological saline containing AN.

The test was continued from the 9th week to the 23rd week. The ordinary feed for rats was freely given to the rats in the S and C groups, and a feed containing 5% spherical carbonaceous adsorbent was freely given to the rats in the A group. The body weight and the amount of urinary protein of each group at the start of the test are shown in Table 1.

After 23 weeks has passed, blood was collected from the jugular vein of each of the rats, and urine was collected for 24 hours. The amounts of the serum creatinine and urinary protein were measured. The results obtained are shown in Table 2.

**Table 1**

| | Body weight (g) | Urinary protein (mg/day) |
|---|---|---|
| S group (n = 6) | 469± 16.5 | 7.2± 2.6 |
| C group (n = 11) | 489± 29.8 | 237± 70 |
| A group (n = 11) | 481± 24.6 | 224± 84 |

| mean ± S.D. | | |
|---|---|---|
| | | |

**Table 2**

| | Serum creatinine (mg/dl) | Urinary protein (mg/day) |
|---|---|---|
| S group (n = 6) | 0.5± 0.1 | 6± 2 |
| | | ]P<0.001 |
| C group (n = 11) | 0.5± 0.1 | 57± 75 |
| | | ]P<0.05 |
| A group (n = 11) | 0.5± 0.1 | 19± 21 |

| mean ± S.D. | | |
|---|---|---|
| | | |

Test for significant difference: Wilcoxon test; P values for the respective groups are described. If there is no significant difference between the groups, the P values are not described.

All the creatinine values of the S, C and A groups were the same as those of normal rats. This shows that the renal function of these rats suffering from nephrotic syndrome was maintained at the normal level.

The amount of urinary protein of the C group began to decrease with time after an elapse of 9 weeks. The decrease in the amount of urinary protein, however, slowed down after 15 weeks had passed, and severe persistent proteinuria appeared. After 23 weeks had passed, the mean value of urinary protein of the C group was 57 mg/day, which was significantly higher than that of the S group. This mean value was also higher than the mean value of urinary protein of 43 mg/day of the C group after 19 weeks had passed. After an elapse of 23 weeks, the body weight of each rat was about 600 g.

Therefore, the mean value of 57 mg/day of rats is converted to 5.7 g/day for humans weighing 60 kg. This value is higher than 3.5 g/day which is the diagnostic criterion for nephrotic syndrome of humans.

On the other hand, the decrease with time in the amount of the urinary protein of the A group between the 9th and 15th weeks was greater than that of the C group.

Thereafter, the urinary protein of the A group did not increase. After 23 weeks had passed, the mean value of urinary protein of the A group was 19 mg/day, which was not significantly different from that of the S group. In the same manner as that described above, this value can be converted to 1.9 g/day for humans. This clealy shows that the amount of urinary protein is lower than the diagnostic criterion of 3.5 g/day for nephrotic syndrome of humans.

The dosage of the spherical carbonaceous adsorbent was about 16g/kg of rat body weight/week, which was calculated on the basis of the feed taken by the rats in the A group.

### Formulation Example 1: Capsule

Two hundred milligrams of the spherical carbonaceous adsorbent obtained in the above Production Example 1 was enclosed in a gelatin capsule to give a capsule.

### Formulation Example 2 : Stick package

Two grams of spherical carbonaceous adsorbent obtained in the above Production Example 1 was put into a stick made of a laminated film (constitution: glassine paper/polyethylene/aluminum foil/polyethylene/polyvinylidene chloride; thickness : 74 ± 8 µm), and was then heat-sealed to obtain a stick package.

Invention also includes in its scope any features which appears to be novel over any prior art from the full preceding description, including the examples which are an integral part of the invention in its broadest definition.

## Claims

**1-** An anti-nephrotic syndrome agent characterized in that it comprises a substantially spherical carbon as an active ingredient in an amount effective for treating nephrotic syndrome.

**2-** An agent according to claim 1, wherein the spherical carbon comprises an activated spherical carbon.

**3-** An agent according to claim 1, wherein the spherical carbon comprises a spherical carbonaceous adsorbent.

**4-** An agent according to anyone of claims 1 to 3, wherein the spherical carbon has a particle diameter ranging from 0.05 to 2 mm.

**5-** An agent according to claim 3 or 4, wherein the spherical carbonaceous adsorbent further has a pore radius of less than 80 angstroms in a pore amount of 0.2 to 1.0 ml/g, a total amount of acidic groups (A) of 0.30 to 1.20 meq/g, a total amount of basic groups (B) of 0.20 to 0.70 meq/g and a ratio of the total amount of acidic groups (A) / total amount of basic groups (B) of 0.40 to 2.5.

**6-** A pharmaceutical composition against nephrotic syndrome, characterized in that it comprises an anti-nephrotic syndrome agent according to anyone of claims 1 to 5 in a dosage form, said agent being optionally combined with a pharmaceutically acceptable excipient or vehicle.

**7-** A pharmaceutical composition against nephrotic syndrome, characterized in that it comprises a substantially spherical carbon as it is in a dosage unit form.

**8-** The composition according to claim 6 or 7, wherein the dosage unit form is a capsule, a stick package, or a divided package.

**9-** Use of a substantially spherical carbon as an active ingredient for the preparation of a pharmaceutical composition for the treatment of nephrotic syndrome.

**10-** Use according to claim 9, wherein the spherical carbon comprises an activated spherical carbon.

**11-** Use according to claim 9, wherein the spherical carbon comprises a spherical carbonaceous adsorbent.

**12-** Use according to anyone of claimq 9 to 11, wherein the spherical carbon has a particle diameter ranging from 0.05 to 2mm.

**13-** Use according to claim 11 or 12, wherein the spherical carbonaceous adsorbent further has a pore radius of less than 80 angstroms in a pore amount of 0.2 to 1.0 ml/g, a total amount of acidic groups (A) of 0.30 to 1.20 meq/g, a total amount of basic groups (B) of 0.20 to 0.70 meq/g and a ratio of the total amount of acidic groups (A) / total amount of basic groups (B) of 0.40 to 2.5.
